# EUROPEAN PATENT APPLICATION

(11) **EP 4 806 491 A1**
(43) Date of publication of application: **16.09.2026**
(21) Application number: 23958319.8
(22) Date of filing: 10.11.2023
(51) Int. Cl.: A61M 25/09

(54) **MEDICAL DEVICE**

(71) Applicant: ASAHI INTECC CO., LTD., Seto-shi, Aichi, 489-0071 (JP)
(72) Inventor: FUKUSHIMA Kensei, Seto-shi, Aichi 489-0071 (JP); IWAMI Takuma, Seto-shi, Aichi 489-0071 (JP)
(74) Representative: Prinz & Partner mbB
(86) International application number: PCT/JP2023/040503
(87) International publication number: WO 2025/099918

(57) **Abstract**

A medical device includes a first region including a distal end, a second region adjacent to the first region on a proximal side of the first region, a third region adjacent to the second region on a proximal side of the second region, and a fourth region adjacent to the third region on a proximal side of the third region. In the second region, when bending reaction force values are acquired at a plurality of measurement points arranged in an axial direction, a product of an average value of the bending reaction force values at the measurement points and a standard deviation of the bending reaction force values is 80 mN² or less.

## Description

### TECHNICAL FIELD

The technology disclosed herein relates to a medical device.

### BACKGROUND ART

Patent Literature 1 discloses a relationship between a measured value of stiffness of a guidewire by a three-point bending test apparatus at a plurality of measurement points and a distance from a distal end of the guidewire to each measurement point.

### CITATION LIST

### Patent Literature

Patent Literature 1: WO 2021/117657 A

### SUMMARY OF INVENTION

### TECHNICAL PROBLEM

Conventional guidewires have a problem that a bending reaction force in a region located relatively on a distal end side is not sufficiently considered. Such a problem is not limited to guidewires but is a problem common to medical devices.

This specification discloses a technology capable of solving the above-described problem.

### SOLUTION TO PROBLEM

The technology disclosed in the present specification can be realized, for example, as the following aspects.

(1) A medical device disclosed in the present specification includes a first region including a distal end, a second region adjacent to the first region on a proximal side of the first region, a third region adjacent to the second region on a proximal side of the second region, and a fourth region adjacent to the third region on a proximal side of the third region. In the second region, when bending reaction force values are acquired at a plurality of measurement points arranged in an axial direction, a product of an average value of the bending reaction force values at each of the measurement points and a standard deviation of the bending reaction force values is 80 mN² or less.
   According to the present medical device, in the second region located relatively on the distal end side, since the product of the average value of the bending reaction force values at each of the measurement points and the standard deviation of the bending reaction force values is relatively small, the bending reaction force values are relatively small and variations in the bending reaction force values are relatively small. Therefore, favorable characteristics can be imparted to the medical device.
(2) In the medical device described above, in the fourth region, when bending reaction force values are acquired at a plurality of measurement points arranged in the axial direction, a square of a Pearson's product-moment correlation coefficient in a relationship between a position of each of the measurement points along the axial direction and the bending reaction force value at that measurement point may be 0.8 or more. According to this configuration, in the fourth region located on the proximal side of the second region, the bending reaction force value increases linearly as a distance from the distal end of the medical device increases. Therefore, a stiffness gap can be suppressed to suppress occurrence of bending of the medical device during pushing, and as a result, vessel selectivity can be improved.
(3) In the medical device described above, the square of the Pearson's product-moment correlation coefficient may be 0.9 or more. According to this configuration, the stiffness gap can be effectively suppressed to effectively suppress occurrence of bending of the medical device during pushing, and as a result, vessel selectivity can be effectively improved.
(4) In the medical device described above, in the fourth region, when bending reaction force values are acquired at a plurality of measurement points arranged in the axial direction, a slope of an approximation line by a least squares method in the relationship between the position of each of the measurement points along the axial direction and the bending reaction force value at that measurement point may be 5 mN/mm or more and 10 mN/mm or less. According to this configuration, in the fourth region located on the proximal side of the second region, the bending reaction force value increases with a relatively large slope as the distance from the distal end of the medical device increases. Therefore, pushability of the medical device can be improved.
(5) In the medical device described above, the slope of the approximation line may be 7 mN/mm or more and 9 mN/mm or less. According to this configuration, pushability of the medical device can be effectively improved.
(6) In the medical device described above, in the fourth region, when bending reaction force values are acquired at a plurality of measurement points arranged in the axial direction, a standard deviation of a rate of change of the bending reaction force values at the measurement points adjacent to each other along the axial direction may be 0.2 or less. According to this configuration, in the fourth region located on the proximal side of the second region, the bending reaction force value changes relatively smoothly as the distance from the distal end of the medical device increases. Therefore, the stiffness gap can be suppressed to suppress occurrence of bending of the medical device during pushing, and as a result, vessel selectivity can be improved.
(7) In the medical device described above, in the fourth region, when bending reaction force values are acquired at a plurality of measurement points arranged in the axial direction, an average value of the rate of change of the bending reaction force values at the measurement points adjacent to each other along the axial direction may be 0.2 or more. According to this configuration, in the fourth region located on the proximal side of the second region, the bending reaction force value increases with a relatively large slope as the distance from the distal end of the medical device increases. Therefore, pushability of the medical device can be improved.

The technology disclosed herein can be realized in various aspects, for example, in aspects such as a medical device and a method for manufacturing the same.

### BRIEF DESCRIPTION OF DRAWINGS

FIG. 1 is an explanatory view schematically illustrating a configuration of a guidewire in an embodiment.
FIG. 2 is an explanatory view illustrating a method for measuring a bending reaction force value of the guidewire.
FIG. 3 is a graph illustrating measurement results of the bending reaction force value.
FIG. 4 is a graph illustrating measurement results of the bending reaction force value.
FIG. 5 is a bar graph illustrating a product of an average value and a standard deviation of the bending reaction force values in a second region.
FIG. 6 is a bar graph illustrating a standard deviation of a rate of change of the bending reaction force values in a fourth region.
FIG. 7 is a bar graph illustrating an average value of the rate of change of the bending reaction force values in the fourth region.
FIG. 8 is an explanatory view illustrating a performance evaluation method for vessel selectivity.
FIG. 9 is an explanatory view illustrating performance evaluation results for vessel selectivity.

### EMBODIMENTS OF THE INVENTION

### A. Embodiment

### (Configuration of Guidewire 100)

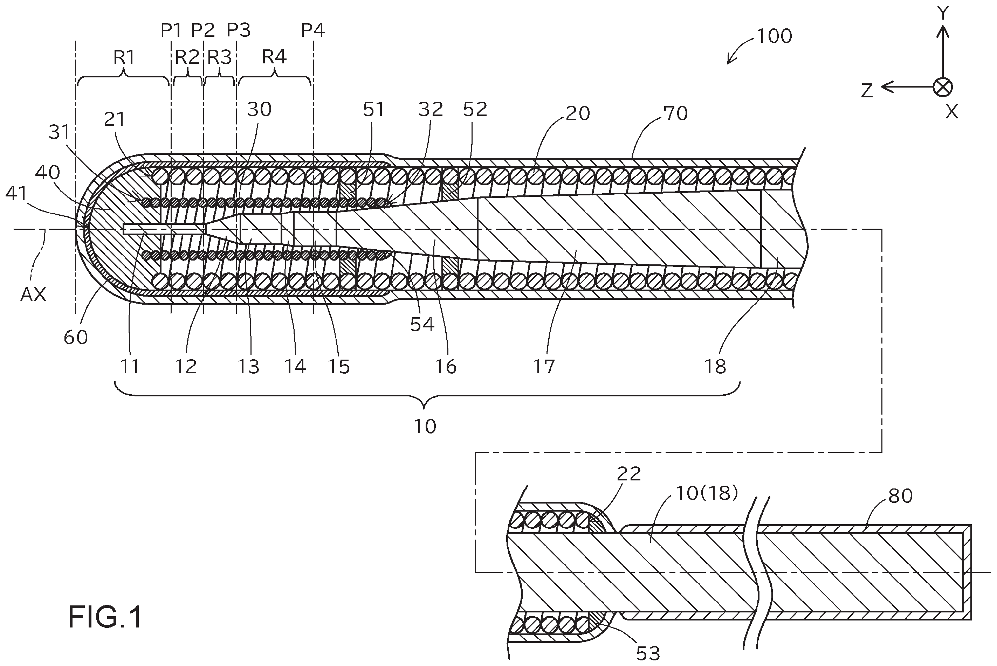

FIG. 1 is an explanatory diagram schematically illustrating a configuration of a guidewire 100 according to an embodiment. FIG. 1 shows XYZ axes orthogonal to one another for specifying directions, and shows a longitudinal section (YZ section) of the guidewire 100. Along a direction parallel to a central axis AX of the guidewire 100 (hereinafter referred to as an "axial direction"), a Z-axis positive direction side is a distal side (distal end side) to be inserted into a body, and a Z-axis negative direction side is a proximal side (proximal end side) to be operated by a physician. FIG. 1 shows a state in which the guidewire 100 has a linear shape substantially parallel to the Z-axis direction as a whole. The guidewire 100 has flexibility to an extent that allows bending. In this specification, regarding the guidewire 100 and each component thereof, an end on the distal side is referred to as a "distal end," the distal end and its vicinity are referred to as a "distal end portion," an end on the proximal side is referred to as a "proximal end," and the proximal end and its vicinity are referred to as a "proximal end portion."

The guidewire 100 is a medical device. The guidewire 100 is inserted into a blood vessel, for example, to guide another medical device (not shown) such as a catheter to a lesion portion in the blood vessel. The guidewire 100 includes a core shaft 10, an outer layer coil 20, an inner layer coil 30, a distal tip 40, a first intermediate joining portion 51, a second intermediate joining portion 52, a first proximal side joining portion 53, a second proximal side joining portion 54, a first coating 60, a second coating 70, and a third coating 80.

The core shaft 10 is an elongated member. A central axis of the core shaft 10 substantially coincides with the central axis AX of the guidewire 100. The core shaft 10 has a first portion 11, a second portion 12, a third portion 13, a fourth portion 14, a fifth portion 15, a sixth portion 16, a seventh portion 17, and an eighth portion 18. The first portion 11, the second portion 12, the third portion 13, the fourth portion 14, the fifth portion 15, the sixth portion 16, the seventh portion 17, and the eighth portion 18 are arranged in this order from the distal end toward the proximal side.

The first portion 11, the third portion 13, the fifth portion 15, and the eighth portion 18 of the core shaft 10 have a constant cross-sectional (XY cross-section) shape at each position along the axial direction. A cross-sectional area of the third portion 13 is larger than a cross-sectional area of the first portion 11. A cross-sectional area of the fifth portion 15 is larger than the cross-sectional area of the third portion 13. A cross-sectional area of the eighth portion 18 is larger than the cross-sectional area of the fifth portion 15. The second portion 12, the fourth portion 14, the sixth portion 16, and the seventh portion 17 of the core shaft 10 smoothly connect cross-sectional shapes of other adjacent portions along the axial direction. The second portion 12, the fourth portion 14, the sixth portion 16, and the seventh portion 17 are tapered portions in which cross-sectional areas gradually increase from the distal end side toward the proximal end side.

The core shaft 10 having such a shape can be manufactured, for example, by performing press working on a precursor having a constant cross section along the axial direction at a press rate corresponding to the shape of each portion of the core shaft 10.

Examples of a forming material of the core shaft 10 include metal materials, and more specifically, stainless steel (SUS302, SUS304, SUS316, etc.), nickel-titanium alloys, piano wire, nickel-chromium alloys, cobalt alloys, tungsten, and the like.

The outer layer coil 20 is a member formed in a hollow cylindrical shape by spirally winding a wire. The outer layer coil 20 is, for example, a densely wound coil. The outer layer coil 20 is disposed so as to surround an outer circumference of the distal end portion of the core shaft 10. In the axial direction, a position of a distal end 21 of the outer layer coil 20 is substantially the same as a position of the distal end of the core shaft 10.

The inner layer coil 30 is a member formed in a hollow cylindrical shape by spirally winding a wire. The inner layer coil 30 is, for example, a densely wound coil. The inner layer coil 30 is disposed so as to surround the outer circumference of the distal end portion of the core shaft 10 in a space inside the outer layer coil 20. In the axial direction, a position of a distal end 31 of the inner layer coil 30 is substantially the same as the position of the distal end of the core shaft 10, and a position of a proximal end 32 of the inner layer coil 30 is on the distal side relative to a position of a proximal end 22 of the outer layer coil 20.

Outer diameters and inner diameters of the outer layer coil 20 and the inner layer coil 30 may be constant along the axial direction, or may change along the axial direction.

Examples of forming materials of the outer layer coil 20 and the inner layer coil 30 include metal materials, more specifically, radiolucent alloys such as stainless steel (SUS302, SUS304, SUS316, etc.), nickel-titanium alloys, piano wire, nickel-chromium alloys, or cobalt alloys, and radiopaque alloys such as gold, platinum, tungsten, or alloys containing these elements (e.g., platinum-nickel alloys).

The distal tip 40 joins the distal end portion of the core shaft 10 and the distal end portions of the outer layer coil 20 and the inner layer coil 30. That is, the outer layer coil 20 and the inner layer coil 30 are connected to the distal tip 40. An outer peripheral surface on the distal end side of the distal tip 40 is a smooth surface (for example, a substantially hemispherical surface). Considering that thicknesses of the first coating 60 and the second coating 70 are very thin, a distal end 41 of the distal tip 40 substantially constitutes the distal end of the guidewire 100. For convenience, the distal end of the guidewire 100 is also referred to as the distal end 41. The first proximal side joining portion 53 joins the core shaft 10 and the proximal end portion of the outer layer coil 20. The second proximal side joining portion 54 joins the core shaft 10 and the proximal end portion of the inner layer coil 30. The first intermediate joining portion 51 joins the core shaft 10 with an intermediate portion (portion excluding the distal end portion and the proximal end portion, the same applies hereinafter) of the outer layer coil 20 and an intermediate portion of the inner layer coil 30. The second intermediate joining portion 52 is located on the proximal side of the first intermediate joining portion 51 and joins the core shaft 10 and the intermediate portion of the outer layer coil 20. Examples of forming materials of the distal tip 40 and each of the joining portions 51, 52, 53, and 54 include metal solders such as silver solder, gold solder, zinc, Sn-Ag alloys, and Au-Sn alloys, and adhesives such as epoxy-based adhesives.

The first coating 60 covers the outer peripheral surface of the distal tip 40 and the outer peripheral surface of the outer layer coil 20 in a region from the distal end 41 of the guidewire 100 to a predetermined position in the axial direction. Examples of a forming material of the coating 60 include, for example, urethane resin.

The second coating 70 covers the outer peripheral surface of the distal tip 40 and the outer peripheral surface of the outer layer coil 20 in the region from the distal end 41 of the guidewire 100 to a predetermined position in the axial direction. More specifically, in a region where the outer peripheral surface of the distal tip 40 and the outer peripheral surface of the outer layer coil 20 are covered with the first coating 60, the second coating 70 covers the outer peripheral surface of the distal tip 40 and the outer peripheral surface of the outer layer coil 20 via the first coating 60 by covering the first coating 60. In a region adjacent to the proximal side of the region, where the outer peripheral surface of the outer layer coil 20 is not covered with the first coating 60, the second coating 70 directly covers the outer peripheral surface of the outer layer coil 20. Such a configuration can be realized, for example, by forming the second coating 70 in a region including the predetermined region after forming the first coating 60 in the predetermined region. The second coating 70 is, for example, a hydrophilic coating. Examples of forming materials of the second coating 70 include polyvinylpyrrolidone, polyacrylic acid, polyacrylamide, polyvinyl alcohol, maleic anhydride copolymers, hyaluronic acid, and the like.

The third coating 80 covers an outer peripheral surface of the core shaft 10 in a region on the proximal side relative to the proximal end 22 of the outer layer coil 20 in the guidewire 100. Examples of a forming material of the third coating 80 include polytetrafluoroethylene (PTFE).

### (Method for measuring bending reaction force value of guidewire 100)

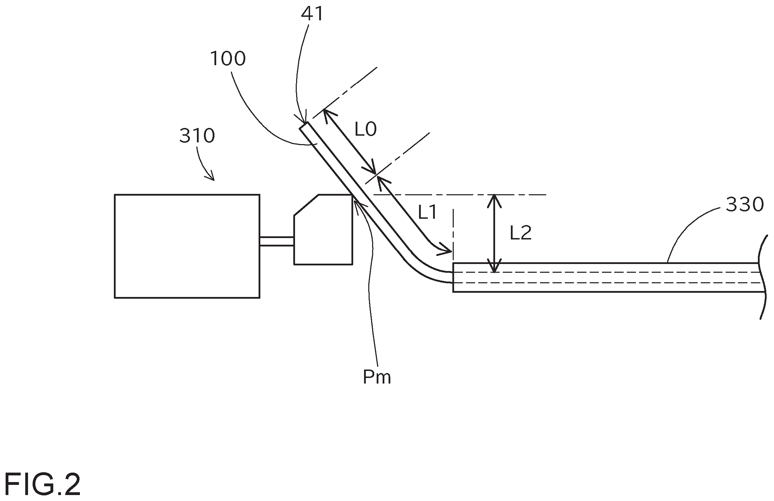

FIG. 2 is an explanatory view illustrating a method for measuring a bending reaction force value of the guidewire 100. When measuring the bending reaction force value at a measurement point Pm at a distance L0 from the distal end 41 of the guidewire 100, an operator inserts the guidewire 100 into a cylindrical body 330 such as a catheter and causes the distal end portion of the guidewire 100 to protrude from a distal end of the cylindrical body 330 into a cantilever state. At this time, a distance L1 from the distal end of the cylindrical body 330 to the measurement point Pm is set to a predetermined distance (for example, 1 mm). The operator presses a force gauge 310 against the measurement point Pm in the guidewire 100 and pushes the guidewire 100 with the force gauge 310 by a predetermined pushing amount L2 (for example, 1 mm) in a direction (up-down direction in the example of FIG. 2) orthogonal to an extension direction of the guidewire 100 (left-right direction in the example of FIG. 2). The operator acquires a load measured by the force gauge 310 at this time as the bending reaction force value at the measurement point Pm of the guidewire 100.

When measuring the bending reaction force value of the guidewire 100, the operator sets the measurement point Pm at a 1 mm pitch and performs measurement, for example, in a region up to 10 mm in the axial direction from the distal end 41 of the guidewire 100, and sets the measurement point Pm at a 2 mm pitch and performs measurement in a region on the proximal side of the region.

### (Characteristics of each region of guidewire 100)

As illustrated in FIG. 1, the guidewire 100 includes a first region R1, a second region R2, a third region R3, and a fourth region R4. The first region R1 is a region including the distal end 41 of the guidewire 100. The second region R2 is a region adjacent to the first region R1 on the proximal side of the first region R1. The third region R3 is a region adjacent to the second region R2 on the proximal side of the second region R2. The fourth region R4 is a region adjacent to the third region R3 on the proximal side of the third region R3. A length along the axial direction of the first region R1 (hereinafter simply referred to as "length") is, for example, 2 mm, a length of the second region R2 is, for example, 6 mm, a length of the third region R3 is, for example, 4 mm, and a length of the fourth region R4 is, for example, 18 mm. In other words, a distance L0 from the distal end 41 of the guidewire 100 to a position P1 of a proximal end of the first region R1 is, for example, 2 mm, a distance L0 from the distal end 41 to a position P2 of a proximal end of the second region R2 is, for example, 8 mm, a distance L0 from the distal end 41 to a position P3 of a proximal end of the third region R3 is, for example, 12 mm, and a distance L0 from the distal end 41 to a position P4 of a proximal end of the fourth region R4 is, for example, 30 mm.

In the present embodiment, in the second region R2 of the guidewire 100, the bending reaction force value is relatively small and variation in the bending reaction force value is relatively small. More specifically, in the second region R2, when bending reaction force values of the guidewire 100 are acquired at a plurality of measurement points Pm arranged in the axial direction, a product of an average value of the bending reaction force values at each of the measurement points Pm and a standard deviation of the bending reaction force values is 80 mN² or less. The product of the average value of the bending reaction force values and the standard deviation of the bending reaction force values may be 70 mN² or less or may be 60 mN² or less.

In the present embodiment, in the fourth region R4 of the guidewire 100, the bending reaction force value increases linearly and with a relatively large slope as the distance L0 from the distal end 41 of the guidewire 100 increases. More specifically, in the fourth region R4, when bending reaction force values of the guidewire 100 are acquired at a plurality of measurement points Pm arranged in the axial direction, the square of the Pearson's product-moment correlation coefficient in the relationship between the position of each of the measurement points Pm along the axial direction and the bending reaction force value at that measurement point Pm is 0.8 or more. The square of the Pearson's product-moment correlation coefficient may be 0.9 or more. A slope of an approximation line by the least squares method in the relationship is 5 mN/mm or more and 10 mN/mm or less. The slope of the approximation line may be 7 mN/mm or more and 9 mN/mm or less.

In the fourth region R4, when the bending reaction force values of the guidewire 100 are acquired at the plurality of measurement points Pm arranged in the axial direction, a standard deviation of a rate of change of the bending reaction force values at the measurement points Pm adjacent to each other along the axial direction is 0.2 or less. The standard deviation of the rate of change may be 0.18 or less. In the fourth region R4, when the bending reaction force values of the guidewire 100 are acquired at the plurality of measurement points Pm arranged in the axial direction, an average value of the rate of change of the bending reaction force values at the measurement points Pm adjacent to each other along the axial direction is 0.2 or more. The average value of the rate of change may be 0.22 or more. A rate of change C1 of the bending reaction force value at a certain measurement point Pm1 is calculated by the following formula (1) using a bending reaction force value V1 at the measurement point Pm1 and a bending reaction force value V0 at a measurement point Pm0 adjacent to the distal end side of the measurement point Pm1. $C 1 = \left(V1/V0\right) - 1$

Each of the characteristics in each region of the guidewire 100 described above can be realized, for example, by appropriately adjusting forming materials of the core shaft 10, a shape of the core shaft 10 (outer diameters and lengths of each portion of the core shaft 10, etc.), forming materials of the outer layer coil 20, a shape of the outer layer coil 20 (an outer diameter, a wire diameter, a winding method, etc. of the outer layer coil 20), forming materials of the inner layer coil 30, a shape of the inner layer coil 30 (an outer diameter, a wire diameter, a winding method, etc. of the inner layer coil 30), and the like. For example, in the second region R2, by making the outer diameter of the core shaft 10 relatively small and making a rate of change of the outer diameter of the core shaft 10 in the axial direction relatively small, at least one of the average value of the bending reaction force values and the standard deviation of the bending reaction force values at each of the measurement points Pm can be made small, and as a result, the product of both can be made small. For example, in the fourth region R4, by making the rate of change of the outer diameter of the core shaft 10 in the axial direction relatively large and making a rate of change of the rate of change relatively small, a value of the square of the Pearson's product-moment correlation coefficient in the relationship between the position of each of the measurement points Pm along the axial direction and the bending reaction force value at that measurement point Pm can be made large, the slope of the approximation line by the least squares method in the relationship can be made in a range of 5 mN/mm or more and 10 mN/mm or less, the standard deviation of the rate of change of the bending reaction force values at the measurement points Pm adjacent to each other along the axial direction can be made small, and the average value of the rate of change of the bending reaction force values at the measurement points Pm adjacent to each other along the axial direction can be made large.

### (Effects of present embodiment)

As described above, the guidewire 100 of the present embodiment includes the first region R1 including the distal end 41, the second region R2 adjacent to the first region R1 on the proximal side of the first region R1, the third region R3 adjacent to the second region R2 on the proximal side of the second region R2, and the fourth region R4 adjacent to the third region R3 on the proximal side of the third region R3. In the second region R2, when the bending reaction force values are acquired at the plurality of measurement points Pm arranged in the axial direction, the product of the average value of the bending reaction force values at each of the measurement points Pm and the standard deviation of the bending reaction force values is 80 mN² or less.

In this way, in the guidewire 100 of the present embodiment, in the second region R2 located relatively on the distal end side, since the product of the average value of the bending reaction force values at each of the measurement points Pm and the standard deviation of the bending reaction force values is relatively small, the bending reaction force values are relatively small and the variation in the bending reaction force values is relatively small. Therefore, favorable characteristics are imparted to the guidewire 100. For example, a risk of vascular perforation by the guidewire 100 can be sufficiently reduced.

In the guidewire 100 of the present embodiment, in the fourth region R4, when the bending reaction force values are acquired at the plurality of measurement points Pm arranged in the axial direction, the square of the Pearson's product-moment correlation coefficient in the relationship between the position of each of the measurement points Pm along the axial direction and the bending reaction force value at that measurement point Pm is 0.8 or more. Therefore, in the fourth region R4 located on the proximal side of the second region R2, the bending reaction force value increases linearly as the distance L0 from the distal end 41 of the guidewire 100 increases. Therefore, the stiffness gap can be suppressed to suppress the occurrence of bending of the guidewire 100 during pushing, and as a result, vessel selectivity can be improved.

In the guidewire 100 of the present embodiment, in the fourth region R4, when the bending reaction force values are acquired at the plurality of measurement points Pm arranged in the axial direction, the slope of the approximation line by the least squares method in the relationship between the position of each of the measurement points Pm along the axial direction and the bending reaction force value at that measurement point Pm is 5 mN/mm or more and 10 mN/mm or less. Therefore, in the fourth region R4 located on the proximal side of the second region R2, the bending reaction force value increases with a relatively large slope as the distance L0 from the distal end 41 of the guidewire 100 increases. Therefore, pushability of the guidewire 100 can be improved.

In the guidewire 100 of the present embodiment, in the fourth region R4, when the bending reaction force values are acquired at the plurality of measurement points Pm arranged in the axial direction, the standard deviation of the rate of change of the bending reaction force values at the measurement points Pm adjacent to each other along the axial direction is 0.2 or less. Therefore, in the fourth region R4 located on the proximal side of the second region R2, the bending reaction force value changes relatively smoothly as the distance L0 from the distal end 41 of the guidewire 100 increases. Therefore, the stiffness gap can be suppressed to suppress the occurrence of bending of the guidewire 100 during pushing, and as a result, vessel selectivity can be improved.

In the guidewire 100 of the present embodiment, in the fourth region R4, when the bending reaction force values are acquired at the plurality of measurement points Pm arranged in the axial direction, the average value of the rate of change of the bending reaction force values at the measurement points Pm adjacent to each other along the axial direction is 0.2 or more. Therefore, in the fourth region R4 located on the proximal side of the second region R2, the bending reaction force value increases with a relatively large slope as the distance L0 from the distal end 41 of the guidewire 100 increases. Therefore, pushability of the guidewire 100 can be improved.

### (Examples)

Three samples (SA1 to SA3) of guidewires having different configurations from each other were prepared, and performance evaluation of each sample was performed.

Tables 1 to 3 are tables illustrating performance evaluation results for the bending reaction force values. In this performance evaluation, bending reaction force values were measured at each measurement point Pm of the guidewire 100 by the measuring method described above (see FIG. 2), and for each region (second region R2 to fourth region R4), an average value A1 of the bending reaction force values, a standard deviation S1 of the bending reaction force values, a product of the average value A1 and the standard deviation S1 (= A1 × S1), a standard deviation S2 of the rate of change of the bending reaction force values, and an average value A2 of the rate of change of the bending reaction force values were calculated. The second region R2 was set as a region where the distance L0 from the distal end 41 of the guidewire 100 is 2 mm or more and 8 mm or less, the third region R3 was set as a region where the distance L0 from the distal end 41 of the guidewire 100 is more than 8 mm and less than 12 mm, and the fourth region R4 was set as a region where the distance L0 from the distal end 41 of the guidewire 100 is 12 mm or more and 30 mm or less.

**[Table 1]**

| | | Sample SA1 | | | | | | |
|---|---|---|---|---|---|---|---|---|
| Region | Distance (L0) from distal end (mm) | Bending reaction force value (mN) | Rate of change of bending reaction force value | Average value (A1) of bending reaction force values (mN) | Standard deviation (S1) of bending reaction force values (mN) | A1*S1 | Standard deviation (S2) of rate of change of bending reaction force values | Average value (A2) of rate of change of bending reaction force values |
| R2 | 2 | 22 | - | 22 | 2.5 | 56 | 0.095 | 0.031 |
| | 3 | 20 | -0.087 | | | | | |
| | 4 | 20 | -0.015 | | | | | |
| | 5 | 19 | -0.025 | | | | | |
| | 6 | 22 | 0.14 | | | | | |
| | 7 | 26 | 0.18 | | | | | |
| | 8 | 26 | -0.0039 | | | | | |
| R3 | 9 | 24 | -0.070 | 25 | 1.6 | 39 | 0.10 | 0.030 |
| | 10 | 27 | 0.13 | | | | | |
| R4 | 12 | 30 | 0.12 | 100 | 50 | 5040 | 0.16 | 0.22 |
| | 14 | 49 | 0.62 | | | | | |
| | 16 | 60 | 0.23 | | | | | |
| | 18 | 61 | 0.020 | | | | | |
| | 20 | 75 | 0.23 | | | | | |
| | 22 | 102 | 0.36 | | | | | |
| | 24 | 124 | 0.21 | | | | | |
| | 26 | 142 | 0.14 | | | | | |
| | 28 | 170 | 0.20 | | | | | |
| | 30 | 184 | 0.084 | | | | | |

**[Table 2]**

| | | Sample SA2 | | | | | | |
|---|---|---|---|---|---|---|---|---|
| Region | Distance (L0) from distal end (mm) | Bending reaction force value (mN) | Rate of change of bending reaction force value | Average value (A1) of bending reaction force values (mN) | Standard deviation (S1) of bending reaction force values (mN) | A1*S1 | Standard deviation (S2) of rate of change of bending reaction force values | Average value (A2) of rate of change of bending reaction force values |
| R2 | 2 | 19 | - | 24 | 8.6 | 211 | 0.35 | 0.20 |
| | 3 | 17 | -0.076 | | | | | |
| | 4 | 23 | 0.37 | | | | | |
| | 5 | 23 | -0.030 | | | | | |
| | 6 | 21 | -0.070 | | | | | |
| | 7 | 24 | 0.12 | | | | | |
| | 8 | 45 | 0.90 | | | | | |
| R3 | 9 | 57 | 0.28 | 63 | 5.3 | 329 | 0.049 | 0.23 |
| | 10 | 68 | 0.18 | | | | | |
| R4 | 12 | 55 | -0.20 | 121 | 34 | 4080 | 0.35 | 0.12 |
| | 14 | 62 | 0.13 | | | | | |
| | 16 | 130 | 1.1 | | | | | |
| | 18 | 126 | -0.038 | | | | | |
| | 20 | 123 | -0.018 | | | | | |
| | 22 | 129 | 0.044 | | | | | |
| | 24 | 122 | -0.050 | | | | | |
| | 26 | 149 | 0.22 | | | | | |
| | 28 | 166 | 0.11 | | | | | |
| | 30 | 145 | -0.13 | | | | | |

**[Table 3]**

| | | Sample SA3 | | | | | | |
|---|---|---|---|---|---|---|---|---|
| Region | Distance (L0) from distal end (mm) | Bending reaction force value (mN) | Rate of change of bending reaction force value | Average value (A1) of bending reaction force values (mN) | Standard deviation (S1) of bending reaction force values (mN) | A1*S1 | Standard deviation (S2) of rate of change of bending reaction force values | Average value (A2) of rate of change of bending reaction force values |
| R2 | 2 | 34 | - | 23 | 4.6 | 105 | 0.13 | -0.066 |
| | 3 | 22 | -0.34 | | | | | |
| | 4 | 20 | -0.098 | | | | | |
| | 5 | 20 | 0.010 | | | | | |
| | 6 | 21 | 0.0049 | | | | | |
| | 7 | 20 | -0.0049 | | | | | |
| | 8 | 21 | 0.029 | | | | | |
| R3 | 9 | 21 | 0.010 | 21 | 0.15 | 3.2 | 0.0023 | 0.012 |
| | 10 | 22 | 0.014 | | | | | |
| R4 | 12 | 29 | 0.33 | 64 | 26 | 1630 | 0.12 | 0.18 |
| | 14 | 34 | 0.17 | | | | | |
| | 16 | 44 | 0.31 | | | | | |
| | 18 | 48 | 0.088 | | | | | |
| | 20 | 51 | 0.058 | | | | | |
| | 22 | 61 | 0.20 | | | | | |
| | 24 | 79 | 0.30 | | | | | |
| | 26 | 97 | 0.22 | | | | | |
| | 28 | 90 | -0.063 | | | | | |
| | 30 | 104 | 0.15 | | | | | |

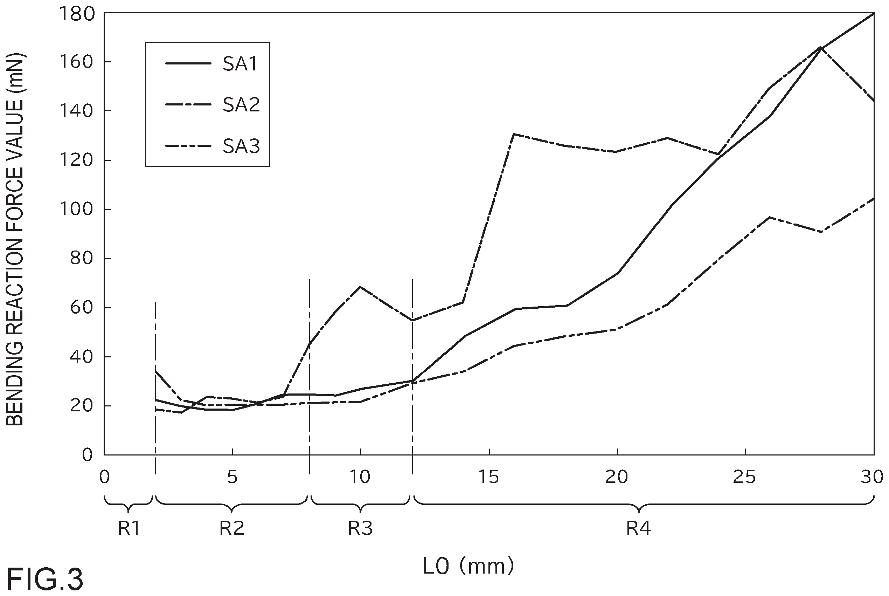

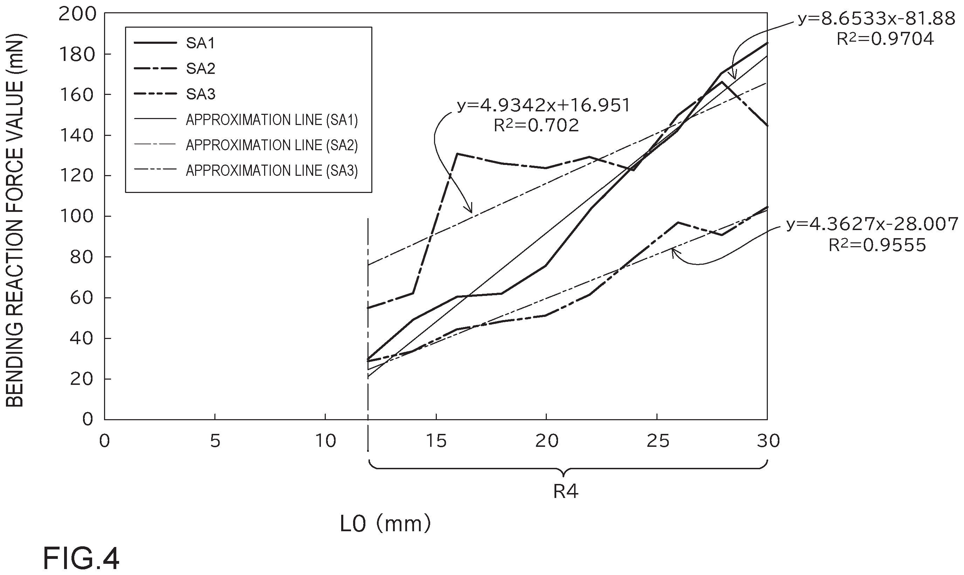

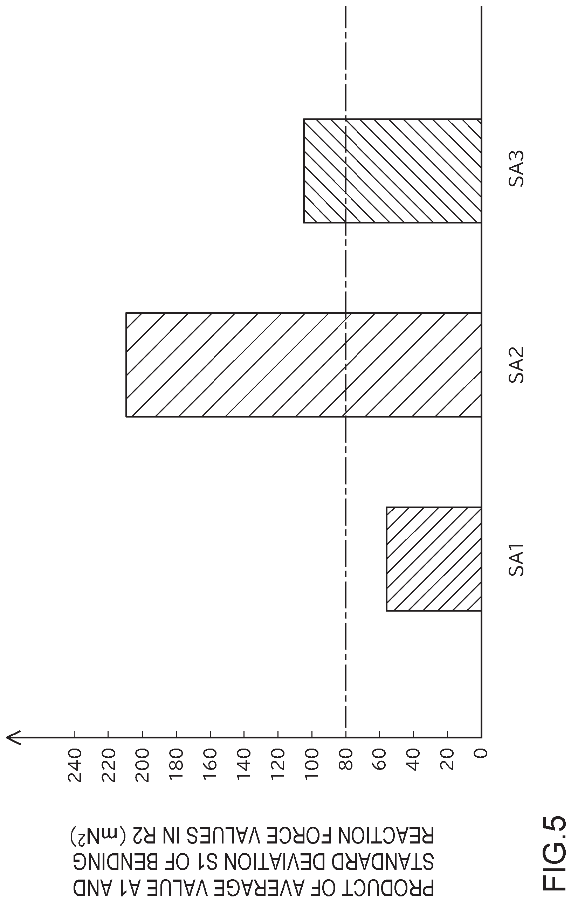

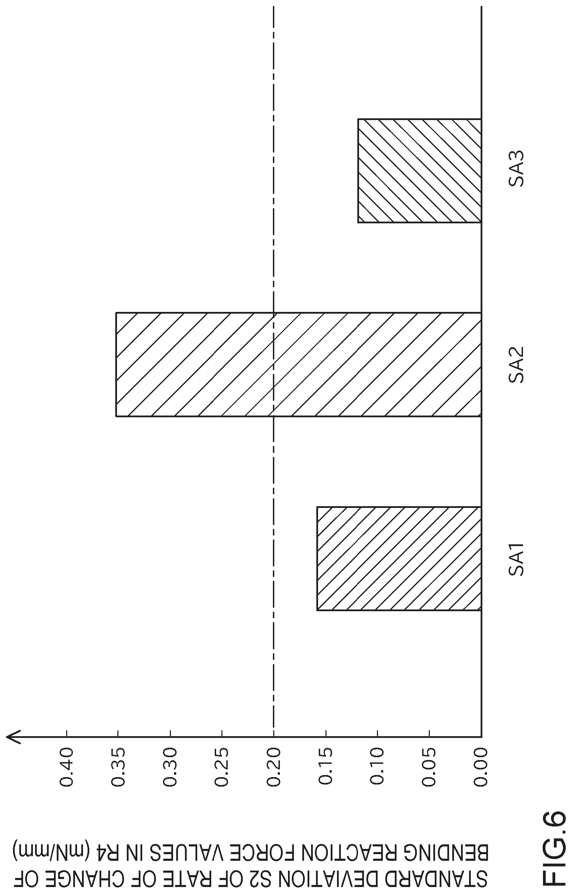

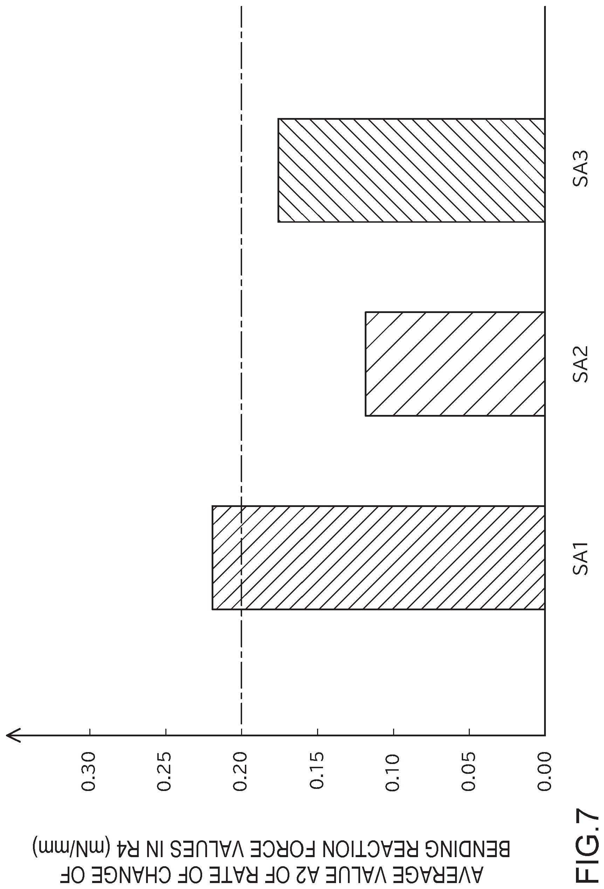

FIGs. 3 and 4 are graphs illustrating measurement results of the bending reaction force value. FIG. 3 illustrates measurement results of the bending reaction force values in the second region R2 to the fourth region R4. FIG. 4 focuses on the fourth region R4 and illustrates approximation lines by the least squares method obtained based on the measurement results in addition to the measurement results of the bending reaction force values. FIG. 5 is a bar graph illustrating a product of the average value A1 and the standard deviation S1 of the bending reaction force values in the second region R2. FIG. 6 is a bar graph illustrating the standard deviation S2 of the rate of change of the bending reaction force values in the fourth region R4. FIG. 7 is a bar graph illustrating the average value A2 of the rate of change of the bending reaction force values in the fourth region R4.

As illustrated in FIG. 3, in sample SA1 (example), compared to samples SA2 and SA3 (comparative examples), the bending reaction force value is relatively small and the variation in the bending reaction force value is relatively small in the second region R2. As a result, as illustrated in FIG. 5, in sample SA1, in the second region R2, the product of the average value A1 of the bending reaction force values at each of the measurement points Pm and the standard deviation S1 of the bending reaction force values is 80 mN² or less. In samples SA2 and SA3, in the second region R2, the product is more than 80 mN².

As illustrated in FIG. 4, in the fourth region R4, in sample SA1, compared to samples SA2 and SA3, the bending reaction force value increases linearly and with a relatively large slope as the distance L0 from the distal end 41 of the guidewire 100 increases. As a result, in sample SA1, in the fourth region R4, the square of the Pearson's product-moment correlation coefficient (R²) in the relationship between the position of each of the measurement points Pm along the axial direction and the bending reaction force value at that measurement point Pm is 0.8 or more, and the slope of the approximation line by the least squares method in the relationship is 5 mN/mm or more and 10 mN/mm or less. In sample SA2, in the fourth region R4, the square of the Pearson's product-moment correlation coefficient (R²) in the relationship described above is less than 0.8. In samples SA2 and SA3, in the fourth region R4, the slope of the approximation line by the least squares method in the relationship described above is less than 5 mN/mm.

As illustrated in FIGs. 6 and 7, in sample SA1, in the fourth region R4, the standard deviation S2 of the rate of change of the bending reaction force values at the measurement points Pm adjacent to each other along the axial direction is 0.2 or less, and the average value A2 of the rate of change is 0.2 or more. In sample SA2, in the fourth region R4, the standard deviation S2 of the rate of change of the bending reaction force values at the measurement points Pm adjacent to each other along the axial direction is more than 0.2. In samples SA2 and SA3, in the fourth region R4, the average value A2 of the rate of change described above is less than 0.2.

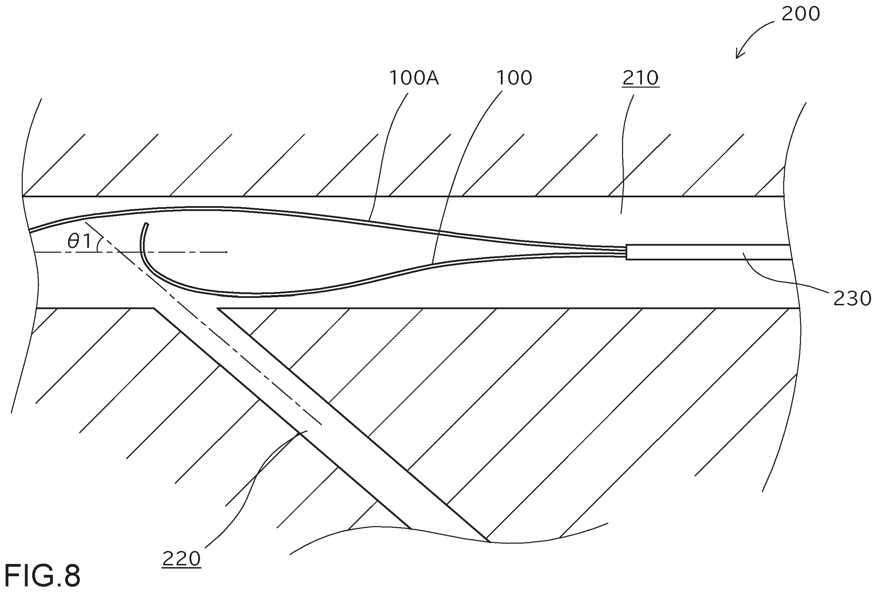

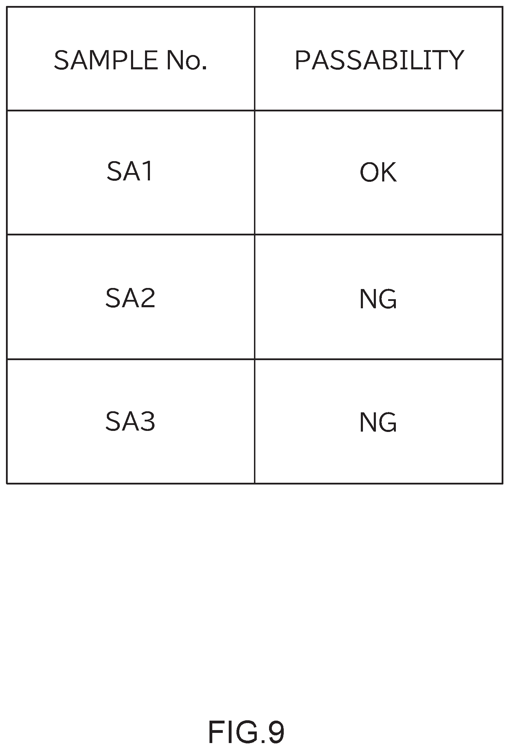

FIG. 8 is an explanatory view illustrating a performance evaluation method for vessel selectivity, and FIG. 9 is an explanatory view illustrating performance evaluation results for vessel selectivity.

As illustrated in FIG. 8, in the performance evaluation for vessel selectivity, a simulated model blood vessel 200 having a main branch 210 with an inner diameter of 4.0 mm and a side branch 220 with an inner diameter of 1.5 mm branching from the main branch 210 so as to turn back at an acute angle with a branching angle θ1 = 35 degrees was used. Using another guidewire 100A inserted through the main branch 210 so as to pass a branching point to the side branch 220 and a double-lumen catheter 230, a guidewire 100 of an evaluation target was inserted into the main branch 210 after shaping a distal end thereof, and whether the guidewire 100 could be guided from the main branch 210 to the side branch 220 and passed through the side branch 220 was evaluated. As illustrated in FIG. 9, in sample SA1 (example), it was possible to pass through the side branch 220. In samples SA2 and SA3 (comparative examples), it was not possible to pass through the side branch 220.

### B. Modifications

The technology disclosed herein is not limited to the above-described embodiment, and can be modified into various modifications without departing from the gist thereof. For example, the following modifications are also possible.

The configuration of the guidewire 100 in the embodiment described above is merely an example and can be variously modified. For example, the guidewire 100 may not include at least one of the outer layer coil 20 and the inner layer coil 30, and the guidewire 100 may include another coil other than the outer layer coil 20 and the inner layer coil 30.

The guidewire 100 may not include at least one of the first coating 60, the second coating 70, and the third coating 80, and the guidewire 100 may include another coating other than the first coating 60, the second coating 70, and the third coating 80.

The technology disclosed herein is applicable not only to the guidewire 100 but also to medical devices in general.

## Claims

1. A medical device (100) comprising:
a first region (R1) including a distal end (41);
a second region (R2) adjacent to the first region (R1) on a proximal side of the first region (R1);
a third region (R3) adjacent to the second region (R2) on a proximal side of the second region (R2); and
a fourth region (R4) adjacent to the third region (R3) on a proximal side of the third region (R3), wherein
in the second region (R2), when bending reaction force values are acquired at a plurality of measurement points (Pm) arranged in an axial direction, a product of an average value (A1) of the bending reaction force values at each of the measurement points (Pm) and a standard deviation (S1) of the bending reaction force values is 80 mN² or less.

2. The medical device (100) according to claim 1, wherein in the fourth region (R4), when bending reaction force values are acquired at a plurality of measurement points (Pm) arranged in the axial direction, a square of a Pearson's product-moment correlation coefficient in a relationship between a position of each of the measurement points (Pm) along the axial direction and the bending reaction force value at that measurement point (Pm) is 0.8 or more.

3. The medical device (100) according to claim 2, wherein the square of the Pearson's product-moment correlation coefficient is 0.9 or more.

4. The medical device (100) according to any one of claims 1 to 3, wherein in the fourth region (R4), when bending reaction force values are acquired at a plurality of measurement points (Pm) arranged in the axial direction, a slope of an approximation line by a least squares method in a relationship between a position of each of the measurement points (Pm) along the axial direction and the bending reaction force value at that measurement point (Pm) is 5 mN/mm or more and 10 mN/mm or less.

5. The medical device (100) according to claim 4, wherein the slope of the approximation line is 7 mN/mm or more and 9 mN/mm or less.

6. The medical device (100) according to any one of claims 1 to 5, wherein in the fourth region (R4), when bending reaction force values are acquired at a plurality of measurement points (Pm) arranged in the axial direction, a standard deviation (S2) of a rate of change (C1) of the bending reaction force values at the measurement points (Pm) adjacent to each other along the axial direction is 0.2 or less.

7. The medical device (100) according to any one of claims 1 to 6, wherein in the fourth region (R4), when bending reaction force values are acquired at a plurality of measurement points (Pm) arranged in the axial direction, an average value (A2) of a rate of change (C1) of the bending reaction force values at the measurement points (Pm) adjacent to each other along the axial direction is 0.2 or more.

8. The medical device (100) according to any one of claims 1 to 7, comprising:
a core shaft (10);
an inner layer coil (30) disposed on an outer peripheral side of a distal end portion of the core shaft (10); and
an outer layer coil (20) disposed on an outer peripheral side of the inner layer coil (30).
